# EUROPEAN PATENT SPECIFICATION

(11) **EP 1 438 003 B1**
(45) Date of publication and mention of the grant of the patent: **13.02.2008**
(21) Application number: 02774635.3
(22) Date of filing: 20.09.2002
(51) Int. Cl.: A61K 8/892, A61Q 5/06

(54) **COSMETIC COMPOSITIONS**
KOSMETISCHE ZUSAMMENSETZUNGEN
COMPOSITIONS COSMETIQUES

(30) Priority: 23.10.2001 GB 0125449
(43) Date of publication of application: 21.07.2004
(73) Proprietor: UNILEVER PLC, London EC4P 4BQ (GB); UNILEVER N.V., 3013 AL Rotterdam (NL)
(72) Inventor: IVANOVA, Katya, Unilever Research Pt. Sunlight, Wirral, Merseyside CH63 3JW (GB); PRATLEY, Stuart K., Unilever Research Pt. Sunlight, Wirral, Merseyside CH63 3JW (GB)
(74) Representative: Tansley, Sally Elizabeth
(86) International application number: PCT/EP2002/010589
(87) International publication number: WO 2003/035017

(56) References cited:
- EP-A- 0 989 153
- EP-A- 1 093 808
- US-A- 4 902 499

## Description

This invention relates to cosmetic compositions comprising a silicone polymer, to a method of styling hair using the polymer and to the use of the polymer to impart softness to hair.

The desire to have the hair retain a particular shape or style is widely held. The most common approach for accomplishing styling of hair is the application of a composition to dampened hair, after shampooing and/or conditioning, or to dry, styled hair. These compositions provide temporary styling benefits and can readily be removed by water or shampooing. To date, the materials employed in hair care compositions to provide styling benefits have generally been natural or synthetic resins and have been applied in the form of, for example, sprays, mousses, gels and lotions.

Style creation products such as hair styling mousses provide human hair with a temporary set which can be removed by water or by shampooing, and function by applying a thin film of a resin or gum onto the hair to adhere adjacent hairs together so that they retain the particular shape or configuration at the time of application.

Cosmetic compositions containing vinyldimethicone/ dimethicone copolymer and a cationic polymer are described in EP-A-1093808. The copolymers described in this document are broadly taught as having a viscosity at 25°C and 0.01Hz of between 1Kpa.s (where 1KPa.s is 1,000 Pa.s) and 100Kpa.s and viscosities at the lower end of this range of 5Kpa.s to 30Kpa.s are preferred. US 4 902 499 discloses silicone polymers for hair conditioning. These polymers are not block copolymers and their viscosity exceeds 1 MPa.s. Aqueous foaming shampoo compositions containing emulsified vinyl silicone polymers are described in our copending application, GB 0102657.4, filed on 2 February 2001. EP 0 989 153 discloses shampoos containing silicone polymers having a viscosity of more than 100 kPa.s, preferably more than 800 kPa.s.

There remains a need for styling compositions having advantages over the compositions of the prior art. For example, it is desirable for styling compositions to give hair greater softness, whilst maintaining or improving the levels of other properties such as, for example, smoothness and ease of comb.

According to the present invention, there is provided a cosmetic composition comprising a silicone polymer having a viscosity at 0.01 Hz and 25°C of greater than 100Kpa.s together with a hair conditioning agent. The composition of this aspect of the invention is not a shampoo composition ie, the composition is not a rinse off cleansing composition comprising from 0.1% to 50% by weight of a foaming surfactant.

In another aspect, the invention provides a cosmetic composition comprising a silicone polymer having a viscosity at 0.01 Hz and 25°C of greater than 100Kpa.s together with from 2% to 30% by weight of an aerosol propellant.

A further aspect of the invention is a method of treating hair which comprises applying to the hair a leave on composition comprising a silicone polymer having a viscosity at 0.01 Hz and 25°C of greater than 100Kpa.s.

A yet further aspect of the invention is the use of a silicone polymer having a viscosity at 0.01 Hz and 25°C of greater than 100Kpa.s for imparting softness to hair.

The present invention is based on the surprising finding that silicone polymers having a viscosity at 0.01 Hz and 25°C of greater than 100Kpa.s have advantages over corresponding polymers having a lower viscosity, when they are used in cosmetic compositions. Given the teaching in the prior art, for example in EP-A-1093808, it could not have been expected that there would be any benefit in using silicone polymers having viscosities greater than 100Kpa.s in cosmetic compositions and certainly no indication that such polymers would have superior properties. For example, it has unexpectedly been found that compositions comprising silicone polymers having viscosities greater than 100Kpa.s can have benefits compared to corresponding compositions containing silicone polymers having lower viscosities, when applied to hair, such as an increased perception of softness of the hair.

Compositions of the invention may have the further surprising advantage of imparting straighter alignment to the hair, compared to compositions containing lower viscosity silicone polymers.

Also, the present invention is at least partly based on the surprising finding that compositions of the invention may provide hair which is both soft and easy to style; those skilled in the art are aware that normally hair becomes more difficult to style as the softness of the hair increases.

Preferably, the silicone polymer is present in compositions of the invention in an amount of from 0.01% to 10% by weight, more preferably 0.1% to 8% by weight, most preferably 0.5% to 4% by weight.

The silicone polymer is preferably a block copolymer comprising a polydimethylsiloxane block eg, ([OSi(Me)₂]ₙ), where Me represents methyl and n is a positive integer of from 5 to 5000. Preferably all the blocks in the co-polymer are silicone based.

The silicone polymer is preferably terminated at at least one end of the polymer chain with an alkenyl group containing from 2 to 6 carbon atoms, for example 2 or 3 carbon atoms, such as a vinyl (CH=CH₂) or 2-propenyl group. The silicone polymer may, however, be terminated with other groups such as hydroxyl, amino or carboxylic acid groups, for example.

The silicone polymer may take a variety of different forms. For example, it may be linear or branched.

Preferably, the silicone polymer comprises at least one group of formula (CH₂-CH₂), bonded at each of its ends to a silicon atom. This group may be formed as a result of the synthetic method that is used to form the polymer.

In one embodiment of the invention, the silicone polymer has the formula:

X{(R¹)(R²) Si- [O-Si(R³) (R⁴)]ₙ-O-Si(R⁵) (R⁶) -CH₂-CH₂}ₚSi(R⁷) (R⁸) [-O-Si (R⁹) (R¹⁰)]ₘ-O-Si(R¹¹) (R¹²) X'

where: X and X' independently represent H or a vinyl (CH=CH₂)group;
R¹, R², R³, R⁴, R⁵, R⁶, R⁷, R⁸, R⁹, R¹⁰, R¹¹ and R¹² independently represent an alkyl group having from 1 to 12 carbon atoms, an aryl group or an aralkyl group;
n and m are each independently positive integers of from 5 to 10000, preferably from 50 to 5000; and
p is a positive integer of from 1 to 100.

Preferably, R¹, R², R³, R⁴, R⁵, R⁶, R⁷, R⁸, R⁹, R¹⁰, R¹¹ and R¹² are all methyl. X and X' are preferably both vinyl.

The term "alkyl" as used herein, includes straight chain and, for alkyl groups containing three or more carbon atoms, branched and also cycloalkyl groups. The cycloalkyl groups may optionally contain a heteroatom selected from nitrogen, oxygen and sulfur. Examples of straight chain alkyl include methyl, ethyl, propyl, butyl, pentyl, hexyl, heptyl, octyl, nonyl, decyl, undecyl, dodecyl, tridecyl and C14, C15, C16, C17 C18, C19 C20, C21 C22, C23 and C24 linear alkyl. Examples of branched alkyl include isopropyl, isobutyl, and tert-butyl. Examples of cycloalkyl groups include cyclopropyl, cyclobutyl, cyclopentyl and cyclohexyl.

The term "aryl" as used herein includes phenyl and other polycyclic fused ring compounds which contain at least one fully aromatic ring, such as, for example, naphthalene.

The term "aralkyl" as used herein refers to C₁ to C₆ alkyl substituted with aryl (eg, benzyl).

The silicone polymer has a viscosity at 0.01 Hz and 25°C of at least 10OKpa.s, preferably from 101Kpa.s to 500Kpa.s, more preferably from 110 to 500Kpa.s, even more preferably from 110 to 300Kpa.s, for example 120 to 190Kpa.s, yet more preferably from 140 to 190Kpa.s. Viscosity is measured as the viscosity of the internal phase of an oil-in-water emulsion of the silicone polymer using a Carri-Med CSL²₅₀₀ Rheometer (TA Instruments).

The silicone polymer is typically supplied in the form of an aqueous oil-in-water emulsion containing an emulsifier (preferably a nonionic emulsifier). The polymer generally constitutes from 50% to 75% by weight of the emulsion. The volume average particle size of the silicone in the emulsion, determined using a Microtrac^{®} UPA150 Ultrafine Particle Analyser (Honeywell, Inc, Industrial Automation and Control, St Petersburg, Florida, USA), will preferably be in the range of from 0.01 µm to 5 µm, more preferably from 0.1 µm to 1.5 µm, most preferably from 0.1 µm to 1 µm. The silicone polymer will preferably have the same particle size in the compositions of the invention and will preferably be in the form of an emulsion in compositions of the invention with the silicone polymer present in the internal phase.

Suitable silicone polymers may be synthesised by known techniques, for example by using metal-catalysed polymerisation from suitable monomers. The silicone polymers may typically be synthesised in an emulsion, with polymerisation occurring in situ within the emulsion droplets. Suitable silicone polymers include those described in EP-A-0874017 (Dow Corning) and may be prepared by the methods described therein. Thus, the silicone may be produced by the reaction of a hydride-terminated silicone with a vinyl-terminated silicone. The hydride-terminated silicone will preferably have a molecular weight of from 186 to 117,000 Da (a viscosity of 0.7 to 65,000 cps) and the vinyl-terminated silicone will typically have a molecular weight of 400 to 62,700 Da (a viscosity of 2 to 10,000 cps). Other suitable methods of forming the silicone polymers include, for example, polycondensation of amine-terminated silicones with carboxylic acid-terminated silicones.
Further methods include that described in EP-A-1069149 (Dow Corning) in which high molecular weight silicones are produced by polycondensation of silanol emulsions using condensation specific acid catalysts such as dialkylsulphonic acid.

The method of the invention involves applying to the hair a composition comprising a silicone polymer having a viscosity at 0.01 Hz and 25°C of greater than 100Kpa.s. Preferably, the method is a method for styling hair and comprises a step of forming the hair into a desired shape or style. The method may involve application of the silicone polymer to the hair in the form of a composition of the invention, although the silicone polymer may be applied to the hair in the form of other compositions.

### Hair conditioning agents

Hair conditioning agents such as hydrocarbons, silicone fluids, and cationic materials, including the cationic surfactant and cationic hair styling polymers described below, may be included in the compositions of the invention. Hair conditioning agents are preferably substances that are capable of spreading onto hair fibres and reducing friction between the fibres. Hair conditioning agents may typically be present in compositions of the invention in amounts of from 0.001% to 10% by weight, preferably 0.1% to 5% by weight. Hair conditioning agents may be single compounds or mixtures of two or more compounds from the same class or different general classes.

Hair conditioning agents may be included in any of the compositions of the invention, regardless of whether they contain a hair styling polymer in addition to the silicone polymer. For example, the compositions of the invention (such as aerosol mousse formulations, for example) may comprise a hair conditioning agent and may be substantially free of hair styling polymer.

Suitable hydrocarbons can be either straight or branched chain and can contain from about 10 to about 16, preferably from about 12 to about 16 carbon atoms. Examples of suitable hydrocarbons are decane, dodecane, tetradecane, tridecane, and mixtures thereof.

Other suitable hair conditioning agents include fatty alcohols and esters of fatty acids and/or esters of fatty alcohols. Examples of such hair conditioning agents are C₈-C₂₄ linear alkyl alcohols (eg, cetyl alcohol, cetearyl alcohol and stearyl alcohol), C₁-C₂₄ linear or branched alkyl esters of C₈-C₂₄ linear alkyl carboxylic acids and C₈-C₂₄ linear alkyl esters of C₁-C₇ carboxylic acids. Isopropyl myristate is an example of a member of this class of hair conditioning agents.

Examples of suitable silicone conditioning agents useful herein can include either cyclic or linear polydimethylsiloxanes, phenyl and alkyl phenyl silicones, and silicone copolyols.

Compositions according to the invention may, optionally, comprise from 0.1% to 10% by weight of a volatile silicone as the hair conditioning agent. Volatile silicones are well known in the art and are commercially available and include, for example linear and cyclic compounds. Volatile silicone oils are preferably linear or cyclic polydimethylsiloxanes containing from about three to about nine silicon atoms.

The compositions of the invention may optionally comprise a cross-linked silicone polymer.

The cross-linked silicone polymer is preferably a non-rigid emulsion-polymerised and may be present in compositions of the invention in an amount of up to 10% by weight based on the total weight of the composition, more preferably from 0.2% to 6% by weight, most preferably from 0.5 to 5% by weight.

Preferred silicone polymers for use in the invention are polydiorganosiloxanes, preferably derived from suitable combinations of R₃SiO_{0.5} units and R₂SiO units where each R independently represents an alkyl, alkenyl (e.g., vinyl), alkaryl, aralkyl, or aryl (e.g. phenyl) group. R is most preferably methyl.

The preferred silicone polymers of the invention are cross-linked polydimethyl siloxanes (which have the CTFA designation dimethicone), and cross-linked polydimethyl siloxanes having end groups such as hydroxyl (which have the CTFA designation dimethiconol). Good results have been obtained with cross-linked dimethiconol.

Cross-linking of the silicone polymer is typically introduced concurrently during emulsion polymerisation of the polymer through the inclusion of the required amount of trifunctional and tetrafunctional silane monomer units, for example, those of formula:
R Si (OH)₃ wherein R represents an alkyl, alkenyl (e.g. vinyl), alkaryl, aralkyl or aryl (e.g. phenyl) group, preferably methyl.

The degree of cross-linking of the silicone polymer can be measured as the percentage of branched monomer units in the silicone polymer and is from 0.05% to 10%, preferably being in the range 0.15% to 7%, e.g. from 0.2% to 2%. Increasing cross-linking is found to improve styling benefits but also to reduce conditioning performance somewhat, so compromise levels must be selected with properties optimised to suit consumer preferences in different cases. Good overall performance has been obtained with dimethiconol 0.3% cross-linked.

Suitable emulsion polymerised cross-linked silicone polymers are commercially available or can be readily made using conventional techniques well known to those skilled in the art.

Cross-linked silicone polymers are described in EP 818190, the contents of which are incorporated herein by reference.

### Cationic Polymer

Hair conditioning agents suitable for use in the invention include cationic polymers.

The cationic polymer, when present in compositions of the invention, is preferably a cationic hair styling polymer and it is preferably present in the composition in an amount of from 0.01% to 10% by weight, more preferably from 0.1% to 10% by weight, most preferably from 0.5% to 5% by weight. Cationic polymers may be used singly or as mixtures of one or more different polymers.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat® FC 370, FC 550, FC 905 and HM-552;
Polyquaternium-46 (prepared by the reaction of vinylcaprolactam and vinylpyrrolidone with methylvinylimidazolium methosulphate), available from BASF as Luviquat®Hold.

### Cationic Surfactant

Hair conditioning agents suitable for use in the invention also include cationic surfactants.

The cationic surfactant, when present in compositions of the invention, is preferably a quaternary ammonium salt having at least one alkyl group containing from 8 to 24 carbon atoms bound to the nitrogen atom. Generally, the cationic surfactant, when present in the compositions of the invention, is present in the composition in an amount of from 0.1% to 5% by weight, more preferably from 0.1% to 2% by weight.

Examples of cationic surfactants include: quaternary ammonium hydroxides, e.g., tetramethylammonium hydroxide, alkyltrimethylammonium hydroxides wherein the alkyl group has from about 8 to 24 carbon atoms, for example octyltrimethylammonium hydroxide, dodecyltrimethylammonium hydroxide, hexadecyltrimethylammonium hydroxide, cetyltrimethylammonium hydroxide, behenyltrimethylammonium hydroxide, octyldimethylbenzylammonium hydroxide, decyldimethyl- benzylammonium hydroxide, stearyldimethylbenzylammonium hydroxide, didodecyldimethylammonium hydroxide, dioctadecyldimethylammonium hydroxide, tallow trimethylammonium hydroxide, cocotrimethylammonium hydroxide, and the corresponding salts thereof, e.g., chlorides, cetylpyridinium hydroxide or salts thereof, e.g., chloride, Quaternium -5, Quaternium -31, Quaternium -18, and mixtures thereof.

### Hair Styling Polymer

Optionally, compositions of the invention may comprise, in addition to the silicone and cationic hair styling polymers mentioned above, one or more other hair styling polymers. Hair styling polymers are well known. Suitable hair styling polymers include commercially available polymers that contain moieties that render the polymers anionic, amphoteric or nonionic in nature. Suitable hair styling polymers include, for example, block and graft copolymers. The polymers may be synthetic or naturally derived.

Examples of suitable naturally-derived polymers include shellac, alginates, gelatins, pectins, cellulose derivatives and chitosan or salts and derivatives thereof. Commercially available examples include Kytamer® (ex Amerchol) and Amaze® (ex National Starch).

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.
The additional styling polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ®ES series available from ISP Corporation esterified copolymers of methyl vinyl ether and maleic anhydride) ;
Luviset PUR® available from BASF.

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and a polyurethane, having a CTFA designation PVP/polycarbamyl polyglycol ester. Suitable carboxylated polyurethane resins are disclosed in EP-A-0619111 and US Patent No. 5,000,955. Other suitable hydrophilic polyurethanes are disclosed in US Patent Nos. 3,822,238; 4,156,066; 4,156,067; 4,255,550; and 4,743,673.

Amphoteric hair styling polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/ acrylates/butylaminoethyl methacrylate copolymer) sold by the National Starch and Chemical Corporation.

Examples of nonionic hair styling polymers are homopolymers of N-vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-0240350 and cross-linked silicone gums such as those described in WO 96/31188.

Also suitable for use as styling polymers in the compositions of the invention are the ionic copolymers described in WO 93/03703, the polysiloxane-grafted polymers disclosed in WO 93/23446, the silicone-containing polycarboxylic acid copolymers described in WO 95/00106 or WO 95/32703, the thermoplastic elastomeric copolymers described in WO 95/01383, WO 95/06078, WO 95/06079 and WO 95/01384, the silicone grafted adhesive polymers disclosed in WO 95/04518 or WO 95/05800, the silicone macro-grafted copolymers taught in WO 96/21417, the silicone macromers of WO 96/32918, the adhesive polymers of WO 98/48770 or WO 98/48771 or WO 98/48772 or WO 98/48776, the graft polymers of WO 98/51261 and the grafted copolymers described in WO 98/51755.

### Surfactant

Optionally, the compositions of the invention may comprise a surfactant, in addition to any cationic surfactant of the type described above. The compositions preferably comprise from 0.01% to 10% by weight of such additional surfactant. The surfactants which are suitable for use in the compositions of the invention may be nonionic, anionic, zwitterionic or a mixture of such surfactants depending on the product form.

The hair styling compositions of the invention preferably comprise a non-ionic surfactant, in an amount of from 0.01% to 10% by weight, preferably from 0.01% to 5% by weight, most preferably from 0.1% to 2% by weight based on total weight.

Examples of suitable non-ionic surfactants are condensation products of aliphatic (C₈-C₁₈) primary or secondary linear or branched chain alcohols or phenols with alkylene oxides, usually ethylene oxide and generally having at least 15, preferably at least 20, most preferably from 30 to 50 ethylene oxide groups. Other suitable non-ionics include esters of sorbitol, esters of sorbitan anhydrides, esters of propylene glycol, fatty acid esters of polyethylene glycol, fatty acid esters of polypropylene glycol, ethoxylated esters, polyoxyethylene fatty ethers and polyoxyethylene fatty ether phosphates.

Of particular use are those non-ionic surfactants of general formula R(EO)ₓ H, where R represents a straight or branched chain alkyl group having an average carbon chain length of 12-18 carbon atoms and x ranges from 30 to 50. Specific examples include steareth-40, steareth-50, ceteareth-30, ceteareth-40, ceteareth-50 and mixtures thereof. Suitable commercially available examples of these materials include Unicol SA-40 (Universal Preserv-A-Chem), Empilan KM50 (Albright and Wilson), NONION PS-250 (Nippon Oils & Fats), Volpo CS50 (Croda Inc), and Incropol CS-50 (Croda Inc).

### Water

Compositions of the present invention will also include water, preferably distilled or de-ionised, as a solvent or carrier for the polymers and other components. Water will typically be present in amounts ranging from 30% to 98%, preferably from 50% to 90% by weight, more preferably from 75% to 90% by weight.

Alcohol may optionally be employed as a co-solvent in compositions of the invention as this can enhance the performance of the styling composition. A suitable alcohol is an aliphatic straight or branched chain monohydric alcohol having 2 to about 4 carbon atoms. Isopropanol and especially ethanol are preferred. A suitable level for the alcohol is up to 20%, preferably from 5% to 15%, by weight based on total weight.

### Product Form

Compositions of the invention are formulated into cosmetic compositions and will comprise cosmetically acceptable components. Compositions of the invention may take a number of product forms such as, for example, compositions intended for application to the hair, including conditioner, styling and colouring products, for example.

Compositions of the invention are preferably leave on products ie, unlike shampoos and conditioners they are not rinsed off the hair after application to the hair.

Preferably, compositions of the present invention are formulated into hair styling compositions which may take a variety of forms, including, for example, hairspray compositions, mousses, gels, lotions, creams, tonics and sprays. These product forms are well known in the art.

Particularly preferred product forms are foaming compositions. Foaming compositions are those compositions which are capable of forming a foam on dispensation from a suitable container, such as a pressurised aerosol container. More preferably, like the compositions of the third aspect of the invention, the compositions of the first and second are in the form of an aerosol mousse. Aerosol hair mousse compositions are emitted from the aerosol container as a foam which is then typically worked through the hair with fingers or a hair styling tool and either left on the hair or rinsed out.

Aerosol-foam compositions of the invention will include an aerosol propellant which serves to expel the other materials from the container, and forms the mousse character in mousse compositions. The aerosol propellant included in styling compositions of the present invention can be any liquefiable gas conventionally used for aerosol containers. Examples of suitable propellants include dimethyl ether and hydrocarbon propellants such as propane, n-butane and iso-butane. The propellants may be used singly or admixed. Water insoluble propellants, especially hydrocarbons, are preferred because they form emulsion droplets on agitation and create suitable mousse foam densities.

The amount of the propellant used is governed by normal factors well known in the aerosol art. For mousses the level of propellant is generally up to 30%, preferably from 2% to 30%, most preferably from 3% to 15% by weight based on total weight of the composition. If a propellant such as dimethyl ether includes a vapour pressure suppressant (e.g. trichloroethane or dichloromethane), for weight percentage calculations, the amount of suppressant is included as part of the propellant.

Preferred propellants are selected from propane, n-butane, isobutane, dimethyl ether and mixtures thereof. Preferably, the propellant comprises dimethyl ether and at least one of propane, n-butane and isobutane.

Aerosol mousse compositions of the invention preferably comprise from 0.1% to 10% by weight, more preferably from 0.1% to 5% by weight, most preferably from 0.1% to 2% by weight, of a nonionic surfactant.

The method of preparing aerosol hair styling mousse compositions according to the invention follows conventional aerosol filling procedures. The composition ingredients (not including the propellant) are charged into a suitable pressurisable container which is sealed and then charged with the propellant according to conventional techniques.

Compositions of the invention may also take a non-foaming product form, such as a hair styling cream or gel. Such a cream or gel will include a structurant or thickener, typically at a level of from 0.1% to 10%, preferably 0.5% to 3% by weight based on total weight.

Examples of suitable structurants or thickeners are polymeric thickeners such as carboxyvinyl polymers. A carboxyvinyl polymer is an interpolymer of a monomeric mixture comprising a monomeric olefinically unsaturated carboxylic acid, and from about 0.01% to about 10% by weight of the total monomers of a polyether of a polyhydric alcohol. Carboxyvinyl polymers are substantially insoluble in liquid, volatile organic hydrocarbons and are dimensionally stable on exposure to air. Suitably the molecular weight of the carboxyvinyl polymer is at least 750,000, preferably at least 1,250,000, most preferably at least 3,000,000. Preferred carboxyvinyl polymers are copolymers of acrylic acid cross-linked with polyallylsucrose as described in US Patent 2,798,053. These polymers are provided by B.F.Goodrich Company as, for example, CARBOPOL 934, 940, 941 and 980. Other materials that can also be used as structurants or thickeners include those that can impart a gel-like viscosity to the composition, such as water soluble or colloidally water soluble polymers like cellulose ethers (e.g. methylcellulose, hydroxyethylcellulose, hydroxypropylmethylcellulose and carboxymethylcellulose), guar gum, sodium alginate, gum arabic, xanthan gum, polyvinyl alcohol, polyvinyl pyrrolidone,hydroxypropyl guar gum, starch and starch derivatives, and other thickeners, viscosity modifiers, gelling agents, etc. It is also possible to use inorganic thickeners such as bentonite or laponite clays.

The hair styling compositions of the invention can contain a variety of non-essential, optional components suitable for rendering the compositions more aesthetically acceptable or to aid use, including discharge from the container, of the product. Such conventional optional ingredients are well known to those skilled in the art, e.g.: preservatives such as benzyl alcohol, methyl paraben, propyl paraben and imidazolidinyl urea; pH adjusting agents such as citric acid, succinic acid, sodium hydroxide and triethanolamine; colouring agents such as any of the FD&C or D&C dyes; perfume oils; chelating agents such as ethylenediamine tetraacetic acid; and polymer plasticising agents such as glycerin and propylene glycol.

The following non-limiting Examples further illustrate the preferred embodiments of the invention. All percentages referred to in the examples and throughout the specification are by weight of active ingredient based on total weight of the composition unless otherwise indicated.

### EXAMPLES

### Example 1

A group of three divinyldimethicone/dimethicone copolymers, listed in the table below, was obtained. The materials were in the form of oil-in-water emulsions with 60% to 65% by weight copolymer content and an emulsifier.

| | Internal viscosity Kpa.s @ 0.01Hz and 25°C (Carri-Med CSL²₅₀₀) * | Particle size µm D50 (Microtrack) |
|---|---|---|
| Comparative Example 1 | 5 to 10 | 0.7 to 0.8 |
| Comparative Example 2 | 50 to 60 | 0.7 to 0.8 |
| Example 1 | 170 | 0.41 |

| | | |
|---|---|---|
| *The viscosity is obtained by doing an oscillation measurement using the following procedure: (i) Pre-experiment step: Parameters: 25 °C Wait for correct temperature perform equilibration eq. duration 5 min wait for zero velocity (ii) Oscillation step: Parameters: frequency ramp Start frequency at 7.958 E-3 Hz End frequency at 40 Hz Temperature: 25 °C controlled variable: displacement ramp mode log applied value (rad) 8.5 E-4 | | |

The viscosity result is taken at 0.01Hz.

The divinyldimethicone/dimethicone copolymers were tested in vitro for the following attributes: smoothness, ease of comb and softness. Treated hair was assessed by 9 experienced pannelists and the results analysed using a statistical program.

| | Smoothness | Ease of Comb | Softness |
|---|---|---|---|
| Comparative Example 1 vs Comparative Example 2 | 48:52 | 31:69 | 46:54 |
| Comparative Example 1 vs Example 1 | 43:57 | 48:52 | 33:67 (significant) |

The high viscosity material provides superior dry conditioning properties, particularly softness, in comparison with the low viscosity ones.

### Examples 2 to 3

The following aerosol mousse formulations were prepared:

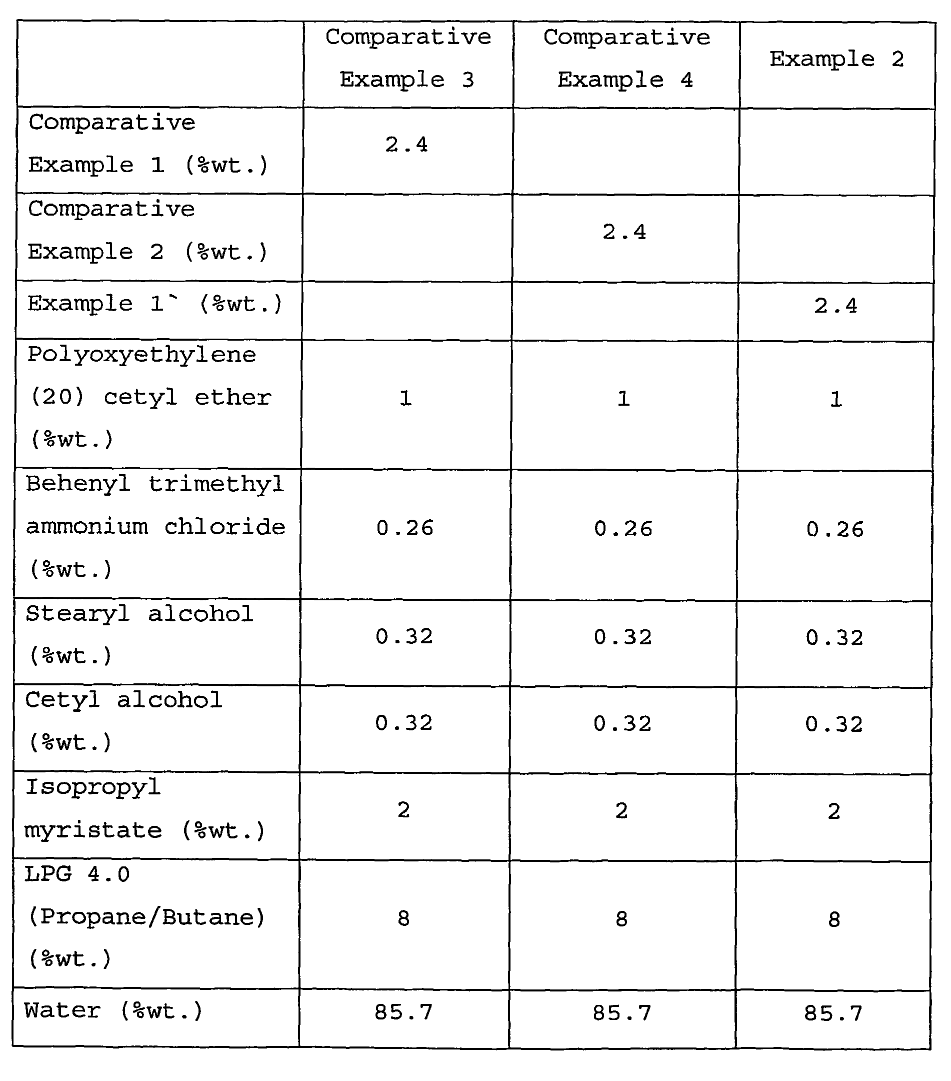

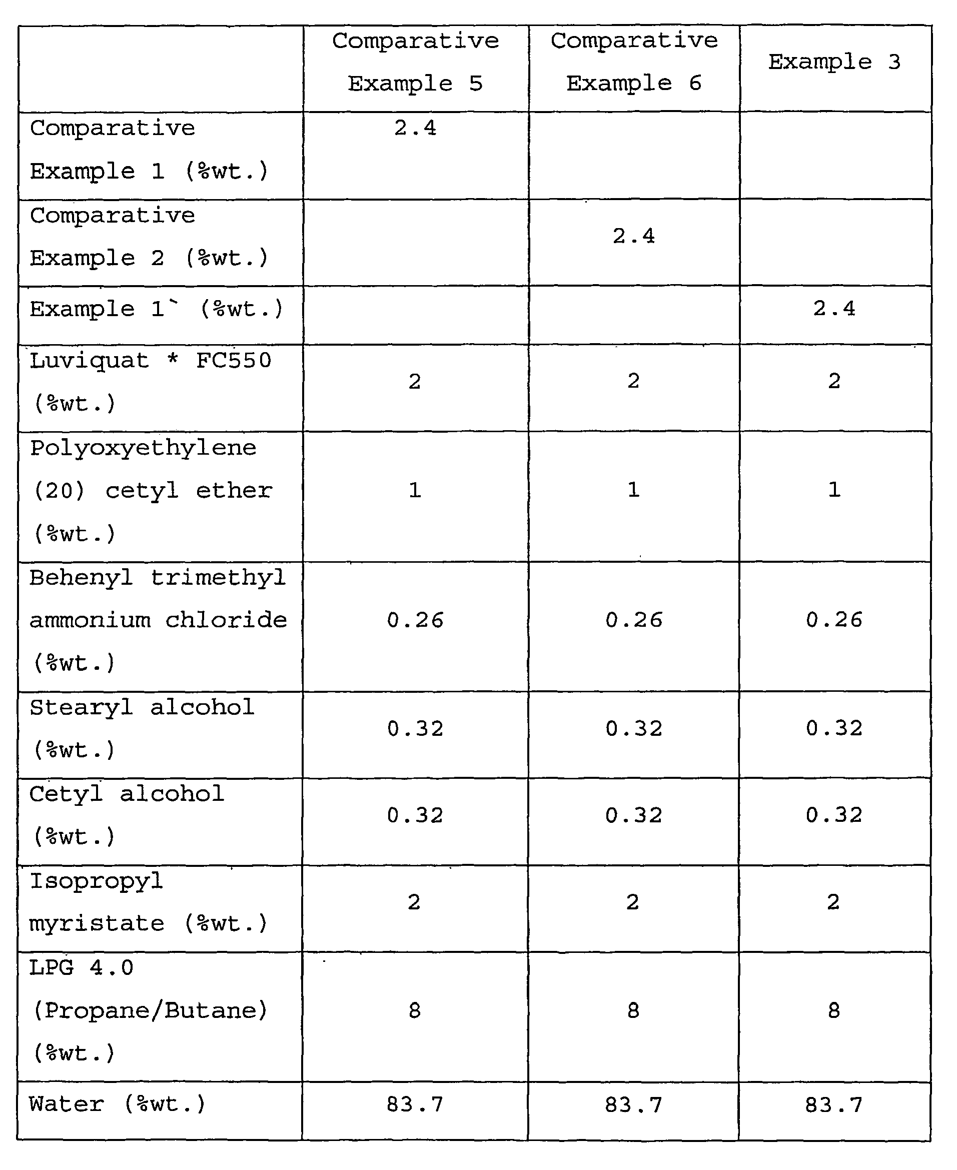

The materials used in Examples 2 to 3 and Comparative Examples 3 to 6 were used as supplied and percentages are by weight based on the active material. The materials were obtained from the following suppliers under the following trade names:

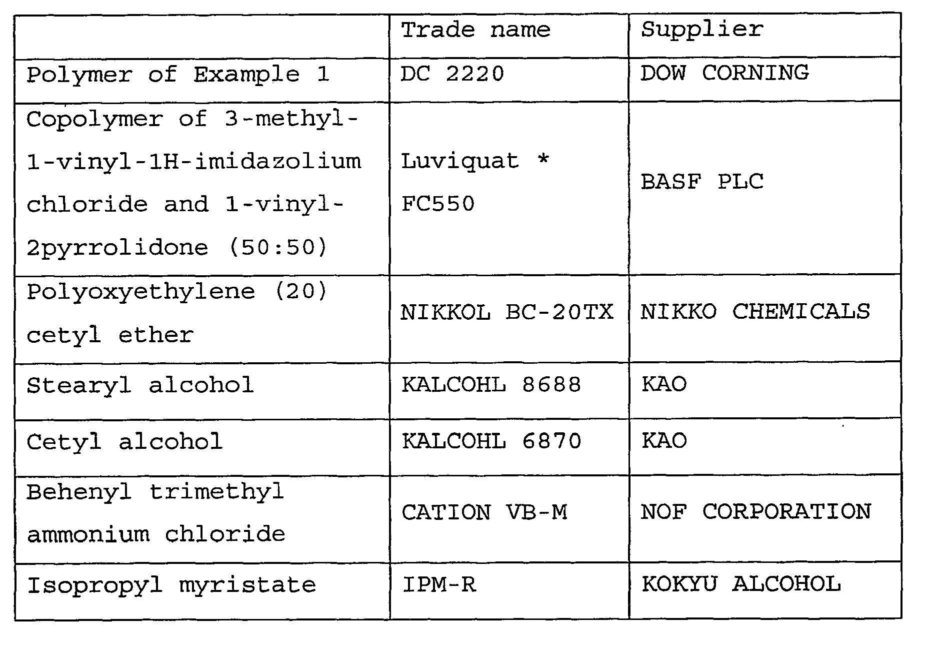

### Example 4

The compositions of Examples 2 and 3 and Comparative Examples 3 to 6 were tested for styling performance in vitro.

A mannequin head with permed Chinese hair (approximately 120g of hair) is washed with 2x5ml 16% by weight shampoo surfactant and then towelled dry until it is no longer dripping wet. 6g of mousse is applied to the hair ensuring even distribution throughout. The hair is then combed straight and left to dry at room temperature for 10 hours, combed and the mannequin head is placed in a humidity chamber at 25°C and 90% relative humidity for 2 hours. The treated hair was assessed by a panel of 10 experienced assessors to determine the treated hair having the straightest alignment of hair.

The results for the compositions of Comparative Examples 3 and 4 and Example 2, where a lower score represents a better alignment, are as follows:

| Composition | Mean Score |
|---|---|
| Example 2 | 1.1 |
| Comparative Example 3 | 3.4 |
| Comparative Example 4 | 3.4 |

Thus, the formulation of Example 2 containing the silicone polymer according to the invention gave straighter alignment.

The results for the compositions of Comparative Examples 5 and 6 and Example 3, where a lower score represents a better alignment, are as follows:

| Composition | Mean Score |
|---|---|
| Example 3 | 1.2 |
| Comparative Example 5 | 2.3 |
| Comparative Example 6 | 2.7 |

Again, the formulation according to the invention gave best alignment.

## Claims

1. Cosmetic leave on composition comprising at least one silicone block copolymer comprising a polydimethyl siloxane group having a viscosity at 0.01 Hz and 25°C from 110 Kpa.s to 500 Kpa. s together with at least one hair conditioning agent, with the proviso that the composition is not a shampoo composition.

2. Cosmetic composition comprising at least one silicone block copolymer comprising a polydimethyl siloxane group having a viscosity at 0.01 Hz and 25°C from 110Kpa.s to 500 Kpa.s together with from 2% to 30% by weight of an aerosol propellant.

3. A composition as claimed in Claim 2 further comprising a hair conditioning agent.

4. A composition as claimed in any one of Claims 1 or 3 when dependent on Claims 1 or 3, wherein the hair conditioning agent comprises a cationic hair styling polymer present in an amount of from 0.01% to 10% by weight and/or a cationic surfactant present in an amount of from 0.1% to 5% by weight.

5. A composition as claimed in any one of Claims 1 to 4, wherein the silicone block co-polymer comprising a polydimethyl siloxane group is present in an amount of from 0.01% to 10 % by weight.

6. A composition as claimed in any one of claims 1 to 5, wherein the silicone block co-polymer comprising a polydimethyl siloxane group is terminated at at least one end of the polymer chain with an alkenyl group containing from 2 to 6 carbon atoms.

7. A composition as claimed in any one of claims 1 to 6, wherein the silicone block co-polymer comprising a polydimethyl siloxane group comprises at least one group of formula (CH₂-CH₂), bonded at each of its ends to a silicon atom.

8. A composition as claimed in Claim 1, wherein the silicone block co-polymer comprising a polydimethyl siloxane group has the formula:
X{(R¹) (R²) Si- [O-Si (R³) (R⁴)]ₙ-O-Si(R⁵)(R⁶) -CH₂-CH₂}ₚSi (R⁷) (R⁸) [-O-Si (R⁹) (R¹⁰)]ₘ-O-Si(R¹¹)(R¹²)X'
where:
X and X' independently represent H or a vinyl (CH=CH₂) group;
R¹, R², R³, R⁴, R⁵, R⁶, R⁷, R⁸, R⁹, R¹⁰, R¹¹ and R¹² independently represent an alkyl group having from 1 to 12 carbon atoms, an aryl group or an aralkyl group;
n and m are each independently positive integers of from 5 to 10000, preferably from 50 to 5000; and p is a positive integer of from 1 to 100.

9. A composition as claimed in any one of Claims 1 to 8, wherein the silicone block co-polymer comprising a polydimethyl siloxane group has a viscosity at 0.01 Hz and 25°C of from 110Kpa.s to 300Kpa.s.

10. A composition as claimed in any one of Claims 1 to 9 which is a hair styling composition.

11. A composition as claimed in Claim 10 which is in the form of an aerosol mousse.

12. A composition as claimed in Claim 11 which comprises from 0.1% to 10% by weight of a nonionic surfactant.

13. A method of treating hair which comprises applying to the hair a leave on composition comprising a silicone block copolymer comprising a polydimethyl siloxane group having a viscosity at 0.01 Hz and 25°C from 110 Kpa.s to 500 Kpa.s.

14. A method as claimed in Claim 13 which is a method for styling hair and which comprises a step of forming the hair into a desired shape or style.

15. A method as claimed in Claim 13 or Claim 14 wherein the silicone polymer is applied to the hair in the form of a composition of any one of Claims 1 to 12.

## Patentansprüche

1. Kosmetische Leave-on-Zusammensetzung, umfassend mindestens ein Silikon-Blockcopolymer, umfassend eine Polydimethylsiloxangruppe mit einer Viskosität bei 0,01 Hz und 25°C von 110 kPa.s bis 500 kPa.s zusammen mit mindestens einem Haar konditionierenden Mittel, mit der Maßgabe, dass die Zusammensetzung keine Shampoozusammensetzung darstellt.

2. Kosmetische Zusammensetzung, umfassend mindestens ein Silikon-Blockcopolymer, umfassend eine Polydimethylsiloxangruppe, mit einer Viskosität bei 0,01 Hz und 25°C von 110 kPa.s bis 500 kPa.s zusammen mit 2 % bis 30 Gewichtsprozent eines Aerosoltreibmittels.

3. Zusammensetzung nach Anspruch 2, weiterhin umfassend ein Haar konditionierendes Mittel.

4. Zusammensetzung nach einem der Ansprüche 1 oder 3, wenn von Ansprüchen 1 oder 3 abhängig, worin das Haar konditionierende Mittel ein kationisches Haarstylingpolymer, das in einer Menge von 0,01 % bis 10 Gewichtsprozent vorliegt, und/oder ein kationisches Tensid, das in einer Menge von 0,1 % bis 5 Gewichtsprozent vorliegt, umfasst.

5. Zusammensetzung nach einem der Ansprüche 1 bis 4, worin das Silikon-Blockcopolymer, umfassend eine Polydimethylsiloxangruppe, in einer Menge von 0,01 % bis 10 Gewichtsprozent vorliegt.

6. Zusammensetzung nach einem der Ansprüche 1 bis 5, worin das Silikon-Blockcopolymer, umfassend eine Polydimethylsiloxangruppe, an mindestens einem Ende der Polymerkette mit einer Alkenylgruppe, enthaltend 2 bis 6 Kohlenstoffatome, beendet ist.

7. Zusammensetzung nach einem der Ansprüche 1 bis 6, worin das Silikon-Blockcopolymer, umfassend eine Polydimethylsiloxangruppe, mindestens eine Gruppe der Formel (CH₂-CH₂), gebunden an jedem von seinen Enden an ein Siliziumatom, umfasst.

8. Zusammensetzung nach Anspruch 1, worin das Silikon-Blockcopolymer, umfassend eine Polydimethylsiloxangruppe, die Formel aufweist:
X{(R¹)(R²)Si-[O-Si(R³)(R⁴)]ₙ-O-Si(R⁵)(R⁶)-CH₂-CH₂}ₚSi(R⁷)(R⁸) [-O-Si (R⁹) (R¹⁰) ]ₘ-O-Si(R¹¹)(R¹²)X',
worin:
X und X' unabhängig H oder eine Vinyl-(CH=CH₂)gruppe wiedergeben;
R¹, R², R³, R⁴, R⁵, R⁶, R⁷, R⁸, R⁹, R¹⁰, R¹¹ und R¹² unabhängig eine Alkylgruppe mit 1 bis 12 Kohlenstoffatomen, eine Arylgruppe oder eine Aralkylgruppe wiedergeben;
n und m jeweils unabhängig positive ganze Zahlen von 5 bis 10 000, vorzugsweise 50 bis 5000, sind; und
p eine positive ganze Zahl von 1 bis 100 ist.

9. Zusammensetzung nach einem der Ansprüche 1 bis 8, worin das Silikon-Blockcopolymer, umfassend eine Polydimethylsiloxangruppe, eine Viskosität bei 0,01 Hz und 25°C von 110 kPa.s bis 300 kPa.s aufweist.

10. Zusammensetzung nach einem der Ansprüche 1 bis 9, die eine Haarstylingzusammensetzung ist.

11. Zusammensetzung nach Anspruch 10, die in Form einer Aerosolmousse vorliegt.

12. Zusammensetzung nach Anspruch 11, die 0,1 % bis 10 Gewichtsprozent eines nichtionischen Tensids umfasst.

13. Verfahren zum Behandeln von Haar, das Auftragen einer Leave-on-Zusammensetzung, umfassend ein Silikon-Blockcopolymer, umfassend eine Polydimethylsiloxangruppe, mit einer Viskosität bei 0,01 Hz und 25°C von 110 kPa.s bis 500 kPa.s auf das Haar umfasst.

14. Verfahren nach Anspruch 13, das ein Verfahren zum Styling von Haar ist und das einen Schritt des Bildens des Haars zu einer gewünschten Form oder zu einem gewünschten Style umfasst.

15. Verfahren nach Anspruch 13 oder Anspruch 14, worin das Silikonpolymer in Form einer Zusammensetzung nach einem der Ansprüche 1 bis 12 auf das Haar aufgetragen wird.

## Revendications

1. Composition cosmétique ne nécessitant pas de rinçage comprenant au moins un copolymère séquencé de silicone comprenant un groupement polydiméthyl siloxane ayant une viscosité à 0,01 Hz et 25°C allant de 110 KPa.s à 500 KPa.s conjointement à au moins un agent de traitement des cheveux, à la condition que la composition ne soit pas une composition de shampooing.

2. Composition cosmétique comprenant au moins un copolymère séquencé de silicone comprenant un groupement polydiméthyl siloxane ayant une viscosité à 0,01 Hz et 25°C allant de 110 KPa.s à 500 KPa.s conjointement à de 2 % à 30 % en poids d'un agent propulseur d'aérosols.

3. Composition selon la revendication 2 comprenant en outre un agent de traitement des cheveux.

4. Composition selon l'une quelconque des revendications 1 ou 3 lorsqu'elle dépend des revendications 1 ou 3, dans laquelle l'agent de traitement des cheveux comprend un polymère cationique mettant en forme les cheveux présent en une quantité allant de 0,01 % à 10 % en poids et/ou un agent tensioactif cationique présent en une quantité allant de 0,1 % à 5 % en poids.

5. Composition selon l'une quelconque des revendications 1 à 4, dans laquelle le copolymère séquencé de silicone comprenant un groupement polydiméthyl siloxane est présent en une quantité allant de 0,01 % à 10 % en poids.

6. Composition selon l'une quelconque des revendications 1 à 5, dans laquelle le copolymère séquencé de silicone comprenant un groupement polydiméthyl siloxane est terminé à au moins une extrémité de la chaîne de polymère avec un groupement alcényle contenant de 2 à 6 atomes de carbone.

7. Composition selon l'une quelconque des revendications 1 à 6, dans laquelle le copolymère séquencé de silicone comprenant un groupement polydiméthyl siloxane comprend au moins un groupement de formule (CH₂-CH₂), lié à chacune de ses extrémités à un atome de silicium.

8. Composition selon la revendication 1, dans laquelle le copolymère séquencé de silicone comprenant un groupement polydiméthyl siloxane est de formule :
X{(R¹) (R²) Si- [O-Si (R³) (R⁴) ]ₙ-O-Si (R⁵) (R⁶) -CH₂-CH₂}_{P} Si (R⁷) (R⁸) [-O-Si (R⁹) (R¹⁰)]ₘ-O-Si (R¹¹) (R¹²) X'
où :
X et X' représentent indépendamment H ou un groupement vinyle (CH=CH₂) ;
R¹, R² , R³, R⁴, R⁵, R⁶, R⁷, R⁸, R⁹, R¹⁰, R¹¹ et R¹² représentent indépendamment un groupement alkyle ayant de 1 à 12 atomes de carbone, un groupement aryle ou un groupement aralkyle ;
n et m sont chacun indépendamment des nombres entiers positifs allant de 5 à 10 000, de préférence de 50 à 5 000 ; et
p est un entier positif allant de 1 à 100.

9. Composition selon l'une quelconque des revendications 1 à 8, dans laquelle le copolymère séquencé de silicone comprenant un groupement polydiméthyl siloxane a une viscosité à 0,01 Hz et 25°C allant de 110 KPa.s à 300 KPa.s.

10. Composition selon l'une quelconque des revendications 1 à 9 qui est une composition pour la mise en forme des cheveux.

11. Composition selon la revendication 10 qui se présente sous la forme d'une mousse d'aérosol.

12. Composition selon la revendication 11 qui comprend de 0,1 % à 10 % en poids d'un agent tensioactif non ionique.

13. Méthode de traitement des cheveux qui comprend l'application sur les cheveux d'une composition sans rinçage comprenant un copolymère séquencé de silicone comprenant un groupement polydiméthyl siloxane ayant une viscosité à 0,01 Hz et 25°C allant de 110 KPa.s à 500 KPa.s.

14. Méthode selon la revendication 13 qui est une méthode pour mettre en forme les cheveux et qui comprend une étape consistant à faire prendre aux cheveux une forme ou un style souhaité.

15. Méthode selon la revendication 13 ou la revendication 14 dans laquelle le polymère de silicone est appliqué sur les cheveux sous la forme d'une composition selon l'une quelconque des revendications 1 à 12.
